# EUROPEAN PATENT APPLICATION

(11) **EP 1 591 010 A2**
(43) Date of publication of application: **02.11.2005**
(21) Application number: 05008195.9
(22) Date of filing: 14.04.2005
(51) Int. Cl.: A01N 35/06, A01N 43/78, A45D 33/34, A47K 7/02, A61L 2/16

(54) **Cosmetic tool having antibacterial property and method for producing the same**

(30) Priority: 15.04.2004 JP 2004120600; 27.05.2004 JP 2004157337; 27.05.2004 JP 2004157351
(71) Applicant: Nakamura, Kenji, Osaka (JP); Nakamura, Koji, Osaka (JP)
(72) Inventor: Nakamura, Kenji, Osaka (JP); Nakamura, Koji, Osaka (JP)
(74) Representative: Vossius & Partner

(57) **Abstract**

An antibacterial cosmetic tool is produced by soaking a base material selected from fiber, yarn, pile fabric, animal hair, wig material, polyurethane sponge and NBR sponge into an aqueous solution containing (A) benzyl ammonium chloride compound and (B) naphthoquinone compound, after which the solution is heated to cause 0.1 to 5.0 percent by weight of (A) benzyl ammonium chloride compound and 0.1 to 5.0 percent by weight of (B) naphthoquinone compound to be adsorbed/contained to/by the material.

## Description

This invention relates to a cosmetic tool offering an excellent antibacterial effect.

The present invention also relates to a cosmetic tool having an antibacterial property with an antibacterial halo width of 5 mm or more maintained even after hand wash by rubbing, made of a material selected from raised woven fabric, raised knit fabric, nonwoven pile and flocked fabric and also of a base material selected from monofilament, animal hair, wig material, polyurethane sponge and NBR sponge.

To be more specific, the present invention relates to a cosmetic tool offering excellent wash resistance and maintaining an excellent antibacterial halo width of 5 mm or more by causing (A) benzyl ammonium chloride compound and (B) naphthoquinone compound to be adsorbed and contained to/by the material by hot solution processing, as well as a method for producing the same.

The cosmetic tool proposed by the present invention includes a cosmetic brush, pile puff, eyelash, wig, porous puff, porous tip, flocked tip, etc.

Representative cosmetic tools include cosmetic brush, pile puff, eyelash, wig, porous puff, porous tip and flocked tip. However, these cosmetic tools tend to generate odor and grow mould due to bacterial growth. Since they come in contact with the skin, there is also a growing demand for cosmetic tools having an antibacterial property so that they can be kept clean and hygienic as much as possible. However, this demand for clean and hygienic cosmetic tools has heretofore remained unanswered, because fixing an antibacterial agent onto the surface of a pile material using a binder decreased the smooth touch. Among other cosmetic tools, it has not been possible to provide an antibacterial puff that offers excellent wash resistance and antibacterial halo effect without reducing the smooth touch or flexibility.

Take cosmetic puffs for example. There are two types of puffs: a sewn puff made of pile fabric, and a porous, elastic puff made of sponge. The former is used for applying cosmetic powder, while the latter is used for applying foundation. Porous, elastic puffs to which an antibacterial property is added by way of soaking a sponge in an aqueous solution of p-hydroxybenzoic acid, parahydroxybenzoate ester, benzoate ester, sodium benzoate, benzalkonium chloride, benzethonium chloride or chlorhexidine gluconate, have been available. However, the aforementioned chemicals would bleed after a single water wash and adding wash resistance to these puffs have not been possible.

To address the above problems, Publication of Unexamined Patent Application No. Sho-60-132504 proposes an antibacterial sponge puff offering wash resistance, wherein said puff is made by mixing one or more of thiabendazole, organic silicone quaternary ammonium salt and 2,4,4-trichloro-2-hydroxyphenyl ether with a sponge material during the sponge production process or by soaking a formed sponge in an antibacterial aqueous solution containing the above chemical(s), and then drying the sponge. However, this sponge could not offer a wash-resistant antibacterial halo effect. A sewn puff made of pile material has many substances and moisture trapped between piles, and it is therefore considered that antibacterial halo is required to achieve an antibacterial effect on a sewn puff.

Publication of Unexamined Patent Application No. Hei-02-116311 describes a cosmetic tool made of a rubber latex sponge that contains a silane coupling agent of. However, this technology could not provide a sewn pile made of pile material that would offer a wash-resistant antibacterial halo effect.

Publication of Unexamined Patent Application No. 2000-000115 discloses a cosmetic applicator formed with animal hair and/or synthetic fiber, which is further plated by 0.05 to 80 percent by weight of silver through electroless plating to obtain an antibacterial cosmetic tool. However, antibacterial treatment with silver ions does not provide an antibacterial halo effect and silver plating causes the material color to turn blackish. Therefore, this technology cannot be used in the production of sewn puffs requiring colors such as white, beige and pink.

Publication of Unexamined Patent Application No. 2000-041730 proposes a cosmetic brush offering an improved antibacterial property, made by impregnating a p-hydroxybenzoate compound into a fiber material comprising nylon, polyester, acrylic or polyurethane, by way of soaking the material in an alcohol solution of the p-hydroxybenzoate compound, which is insoluble in water, and thus allowing the material to absorb the p-hydroxybenzoate compound. However, this technology does not provide any halo effect. To achieve a wash-resistant antibacterial halo effect, in particular, a technology must be developed that allows adsorption in a sustained-release manner using a water-soluble antibacterial agent.

As explained above, in view of the fact that there have been no cosmetic tools given excellent wash-resistant antibacterial treatment, which are made e.g. of a base material selected from raised woven fabric, raised knit fabric, nonwoven pile and flocked fabric and also of a base material selected from monofilament, animal hair, wig material, polyurethane sponge and NBR sponge, the inventors studied arduously to solve this problem and succeeded in providing an antibacterial cosmetic tool that offers an excellent antibacterial property and maintains an antibacterial halo width of 5 mm or more even after hand wash by rubbing, without changing the feel and touch of the pile material. The cosmetic tool proposed by the present invention includes a cosmetic puff as well as cosmetic brush, pile puff, eyelash, wig, porous puff, porous tip or flocked tip.

The present invention basically encompasses the following configurations:
(1) A cosmetic tool having an antibacterial property with an antibacterial halo width of 5 mm or more maintained even after hand wash by rubbing, obtainable by causing 0.1 to 5.0 percent by weight of (A) benzyl ammonium chloride compound and 0.1 to 5.0 percent by weight of (B) naphthoquinone compound to be adsorbed, through hot solution processing, to the cosmetic tool.
(2) A cosmetic tool as described in Item 1, obtainable by causing (A) benzyl ammonium chloride compound and (B) naphthoquinone compound to be adsorbed, through hot solution processing and via a silane coupling agent, to the cosmetic tool.
(3) A cosmetic tool as described in Item 1 or 2, wherein the cosmetic tool is selected from a cosmetic brush, pile puff, porous puff, porous tip, flocked tip, eyelash or wig.
(4) A cosmetic tool as described in any one of Items 1 to 3, wherein the base material of the cosmetic tool is selected from animal hair, synthetic fiber, monofilament, raised woven fabric, raised knit fabric, nonwoven pile, flocked fabric, wig material, polyurethane sponge or NBR sponge.
(5) A cosmetic tool as described in Item 4, wherein the synthetic fiber is selected from at least one or more fibers selected from the group consisting of nylon, PBT (polybutylene terephthalate), PET (polyethylene terephthalate), acrylic and modacrylic; or a sheath-core fiber having a surface layer formed by hydrophilic polymer and an inside formed by hydrophobic polymer.
(6) A method for producing a cosmetic tool having an antibacterial property with an antibacterial halo width of 5 mm or more maintained even after hand wash by rubbing, characterized by soaking the cosmetic tool in an aqueous solution containing (A) benzyl ammonium chloride compound and (B) naphthoquinone compound, and applying hot solution processing.
(7) A method for producing a cosmetic tool as described in Item 6, wherein the base material of the cosmetic tool is selected from animal hair, synthetic fiber, monofilament, raised woven fabric, raised knit fabric, nonwoven pile, flocked fabric, wig material, polyurethane sponge or NBR sponge.
(8) A method for producing a cosmetic tool as described in Item 6 or 7, characterized by applying hot solution processing to the cosmetic tool in an aqueous solution containing (A) benzyl ammonium chloride compound and (B) naphthoquinone compound to which a carrier is added.
(9) A method for producing a cosmetic tool as described in Item 8,
wherein benzoic acid, methyl p-hydroxybenzoate, ethyl p-hydroxybenzoate, butyl p-hydroxybenzoate, bisphenol A, bisphenol B, 2-hydroxybisphenol, p-biphenylamine, p-aminosalicylic acid or orthophenyl phenol is used as the carrier.

The adsorbed amount of (A) and (B) can be obtained from the rate of weight increase of the adsorbing material, e.g. the pile material, in dry condition after the processing, with respect to the weight before the processing.

As the benzyl ammonium chloride compound (A), ethylbenzalkonium chloride, lauryl dimethyl benzyl ammonium chloride, cetyl dimethyl benzyl ammonium chloride or dodecyl benzyl ammonium chloride can be used, among others.

As the naphthoquinone compound (B), 1,4-naphthoquinone,
2-hydroxy-3-chloronaphthoquinone, 2,3-dichloro-1,4-naphthoquinone,
2-alkoxy-3-chloronaphthoquinone, naphtho[2,3-D]thiazole-4,9-dion or
2-alkyl-2,3-thiazole-4,9-dion can be used, among others.

Regarding the cosmetic tool proposed by the present invention , the base material, e.g. the pile fabric to be used for a cosmetic brush or pile puff may be raised woven fabric; raised knit fabric, nonwoven pile and flocked fabric. For flocked fabric and a flocked tip, the material fibers can be in the state of cut fiber for flocking. For a porous puff or porous tip, polyurethane sponge or NBR (nitrile butadiene rubber) sponge can be used. Animal hair and monofilament are available for making eyelashes, while wig material can be used for making wigs. It is also effective to use a carrier when adsorbing constituents (A) and (B) to these materials through hot solution processing. The aforementioned carrier may be benzoic acid, methyl p-hydroxybenzoate, ethyl p-hydroxybenzoate, butyl p-hydroxybenzoate, bisphenol A, bisphenol B, 2-hydroxybisphenol, p-biphenylamine, p-aminosalicylic acid, orthophenyl phenol, or cyanoethylbenzyl ether. Both constituents (A) and (B) should be added by 0.05 to 0.15 percent on the weight of fiber in the hot solution processing.

One basic requirement of the present invention is to use the aforementioned constituents (A) and (B) together, instead of using only one of them. This results in a cosmetic tool offering excellent wash resistance and antibacterial halo effect.

The cosmetic tool proposed by the present invention provides an antibacterial property without changing the natural touch of the fiber used, by way of soaking a base material selected e.g. from pile fabric, fiber, animal hair, wig material, polyurethane sponge and NBR into an aqueous solution containing (A) and (B), and then heating the solution to cause (A) and (B) to be adsorbed and fixed to the material, just like when dyeing the material. This antibacterial property is such that the cosmetic tool offers excellent rubbed hand-wash resistance and maintains an antibacterial halo width of 5 mm or more even after such washing. However, a satisfactory level of wash resistance cannot be obtained if only constituent (A) or (B) is adsorbed to the material through hot solution processing.

If the adsorbed amounts of constituents (A) and (B) are both 0.1 percent by weight or less (based on the weight of fiber), a sufficient halo effect cannot be obtained. If the adsorbed amounts are 5 percent by weight or more, the obtained halo effect will not increase proportionally to the adsorptions, which is not economically desirable.

If the aforementioned hot-solution adsorption method does not achieve sufficient adsorption of constituents (A) and (B) due to the type of fiber or the crystallization or polymerization degree of synthetic fibers, then constituents (A) and (B) can be adsorbed and fixed via a silane coupling agent.

For example, γ-glycidoxypropyl trimethoxysilane, γ-methacryloxypropyl trimethoxysilane, γ-mercaptopropyl trimethoxysilane, γ-(2-aminoethyl)aminopropyl trimethoxysilane or vinyl triacetoxysilane may be used as this silane coupling agent.

The aforementioned pile fabric, fiber, animal hair, wig material, polyurethane sponge or NBR can be processed even in the state of cut fiber, monofilament or yarn, and this processing can be performed in a manner similar to dyeing, or simultaneously with dyeing.

The materials that can be used as the fiber material include, but are not limited to, the following polymer materials: cotton, rayon, nylon, PBT (polybutylene terephthalate), PET (polyethylene terephthalate), PTT (polytrimethylene terephthalate), acrylic and modacrylic.

Of the fibers, a sheath-core fiber having a surface layer formed by hydrophilic polymer and an inside formed by hydrophobic polymer provides a functional elasticity suitable for a brush, offers an excellent liquid-retention property, and embodies an ideal puff function. A synthetic fiber having an ethylene PVA copolymer sheath and a PET core provides a better puff function than other synthetic fibers. One example of this type of synthetic fiber is "Sophista" (registered trademark; manufactured by Kuraray).

The rubbed hand-wash resistance of the present invention was evaluated after 100 hand washes by rubbing following a 30-minute soak at 40°C in a water containing neutral detergent solution by 0.1 percent by weight, in accordance with JIS-L-1042.

The antibacterial test measured the growth inhibition width (halo width) in accordance with the test defined in JIS-L-1902. Since generation of halo is based on bleed-out of antibacterial agent, it became more difficult to obtain a wash resistance property when the amount of elution was greater. The present invention is characterized by fixing an antibacterial agent to a pile material via adsorption by hot solution processing, just like dyeing, thereby allowing the agent to bleed gradually.

While conventional products failed to embody a rubbed hand-wash resistance equivalent to a halo width of 5 mm or more, the present invention maintains a halo width of 7 to 8 mm even after wash.

Before, no cosmetic tools given an antibacterial treatment offering excellent wash resistance were available among cosmetic brushes, pile puffs, eyelashes, wigs, porous puffs, porous tips, flocked tips, etc. The present invention provides an antibacterial cosmetic tool, such as a puff, that maintains an antibacterial halo width of 5 mm or more even after hand wash by rubbing, without changing the feel or touch of the puff.

As explained above, cosmetic tools such as cosmetic brushes, pile puffs, eyelashes, wigs, porous puffs, porous tips and flocked tips are subject to significant bacterial growth. Since they are used on the face, the market has been keenly demanding, in vain, antibacterial, wash-resistant products to ensure cleanliness. The present invention provides an antibacterial property that is resistant to wash, and the halo test clearly showed a retained halo width of 5 mm or more. As a result, the present invention can prevent the problems of conventional products arising from bacterial growth, such as odor and mould.

In the present invention, the hot solution processing is preferably carried out in a dyeing pot used for dyeing fibers. It is preferably carried out at temperatures of 80°C or above, or more preferably at 100°C or more, and even more preferably at 120°C. The process may be performed e.g. simultaneously with dyeing, or after dyeing. The method proposed by the present invention can be used for making cosmetic tools such as cosmetic brushes, pile puffs, eyelashes, wigs and porous, elastic puffs.

The present invention is explained in details using examples.

### Example 1

A pile material was soaked in each of the processing solutions shown in Table 1, based on the solution volume of 15 times the weight of the material for cosmetic tool (bath ratio of 1:15). The solution was heated to 96°C for 60 minutes, after which the material was washed in water and then dried.

As the silane coupling agent, an aqueous solution containing γ-methacryloxypropyl trimethoxysilane (product number SH6030, manufactured by Toray Silicone) by 1 percent by weight was prepared and processed at 70°C for 60 minutes.

The halo test against bacteria in the pile material was conducted using Staphylococcus Aureus in accordance with JIS-L-1902.

Next, the wash-resistance test was conducted in accordance with JIS-L-1042, where 100 hand washes by rubbing following a 30-minute soak in a 40°C wash-test solution were repeated twice, after which the material was washed in water and then dried.

**Table 1**

| Compositions of Processing Solutions | | |
|---|---|---|
| | Compound(s) | Compound concentration |
| Processing solution (1) | Lauryl trimethyl ammonium chloride (manufactured by Kao) | 1.000 wt% |
| | Naphtho[2,3-D]thiazole-4,9-dion (manufactured by Kawasaki Kasei Chemicals) | 1.000 wt% |
| Processing solution (2) | Lauryl trimethyl ammonium chloride (manufactured by Kao) | 2.000 wt% |
| | Naphtho[2,3-D]thiazole-4,9-dion (manufactured by Kawasaki Kasei Chemicals) | 2.000 wt% |
| Processing solution (3) | Cetyl dimethyl benzyl ammonium chloride (manufactured by Kao) | 1.000 wt% |
| | 2,3-dichloronaphthoquinone (manufactured by Kawasaki Kasei Chemicals) | 1.000 wt% |
| Processing solution (4) | Cetyl dimethyl benzyl ammonium chloride (manufactured by Kao) | 2.000 wt% |
| | 2,3-dichloronaphthoquinone (manufactured by Kawasaki Kasei Chemicals) | 2.000 wt% |
| Processing solution (5) | Lauryl trimethyl ammonium chloride (manufactured by Kao) | 0.095 wt% |
| | Naphtho[2,3-D]thiazole-4,9-dion (manufactured by Kawasaki Kasei Chemicals) | 0.095 wt% |
| Processing solution (6) | Cetyl dimethyl benzyl ammonium chloride (manufactured by Kao) | 0.095 wt% |
| | 2,3-dichloronaphthoquinone (manufactured by Kawasaki Kasei Chemicals) | 0.095 wt% |
| Processing solution (7) | Lauryl trimethyl ammonium chloride (manufactured by Kao) | 2.000 wt% |
| Processing solution (8) | Naphtho[2,3-D]thiazole-4,9-dion (manufactured by Kawasaki Kasei Chemicals) | 2.000 wt% |
| Processing solution (9) | Cetyl dimethyl benzyl ammonium chloride (manufactured by Kao) | 2.000 wt% |
| Processing solution (10) | 2,3-dichloronaphthoquinone (manufactured by Kawasaki Kasei Chemicals) | 2.000 wt% |

A total of nine materials and base materials were used in the examples and comparative examples. They are: (1) cotton, (2) horse hair, (3) acrylic, (4) rayon, (5) nylon, (6) polyurethane, (7) NBR, (8) PET, and (9) PBT.

The halo test results are shown in Tables 2 and 3.

**Table 2**

| Example | Processing solution | Cosmetic tool / Material/base material | Silane coupling | Adsorption (wt%) | Halo width before wash | Halo width after wash |
|---|---|---|---|---|---|---|
| Ex. 1-1 | (1) | Pile puff / Cotton velvet | Not used | 0.7 | 5 mm | 5 mm |
| | | | Used | 1.7 | 6 | 6 |
| Ex. 1-2 | (2) | Eyelash / Horse hair | Not used | 1.5 | 6 | 6 |
| | | | Used | 2.6 | 7 | 7 |
| Ex. 1-3 | (3) | Wig / Acrylic wig | Not used | 0.8 8 | 5 | 5 |
| | | | Used | 2.3 | 6 | 6 |
| Ex. 1-4 | (4) | Pile puff / Rayon velvet | Not used | 1.7 | 6 | 6 |
| | | | Used | 2.7 | 7 | 7 |
| Ex. 1-5 | (1) | Pile puff / Nylon tricot pile | Not used | 0.7 | 5 | 5 |
| | | | Used | 1.6 | 6 | 6 |
| Ex. 1-6 | (2) | Pile puff / Nylon tricot pile | Not used | 0.8 | 5 | 5 |
| | | | Used | 2.0 | 6 | 6 |
| Ex. 1-7 | (3) | Porous puff / Polyurethane sponge | Not used | 0.8 | 5 | 5 |
| | | | Used | 1.7 | 6 | 6 |
| Ex. 1-8 | (4) | Porous tip / NBR sponge | Not used | 1.0 | 6 | 6 |
| | | | Used | 2.0 | 7 | 7 |
| Ex. 1-9 | (1) | Pile puff / PET circular knit pile | Not used | 0.4 | 5 | 5 |
| | | | Used | 1.1 | 6 | 6 |
| Ex. 1-10 | (2) | Pile puff / PET circular knit pile | Not used | 0.5 | 6 | 6 |
| | | | Used | 1.7 | 7 | 7 |
| Ex. 1-11 | (3) | Eyelash / Horse hair | Not used | 0.6 | 5 | 6 |
| | | | Used | 1.3 | 6 | 7 |
| Ex. 1-12 | (4) | Wig / Acrylic wig | Not used | 0.6 | 6 | 6 |
| | | | Used | 1.7 | 7 | 7 |
| Ex. 1-13 | (1) | Pile puff / PBT circular knit pile | Not used | 0.5 | 6 | 6 |
| | | | Used | 1.4 | 7 | 7 |
| Ex. 1-14 | (2) | Pile puff / PBT circular knit pile | Not used | 0.6 | 6 | 6 |
| | | | Used | 1.8 | 7 | 7 |
| Ex. 1-15 | (3) | Pile puff / Acrylic circular knit pile | Not used | 0.6 | 6 | 6 |
| | | | Used | 1.5 | 7 | 7 |
| Ex. 1-16 | (4) | Pile puff/ Acrylic circular knit pile | Not used | 0.5 | 6 | 6 |
| | | | Used | 1.9 | 7 | 8 |

**Table 3**

| Comparative example | Processing solution | Cosmetic tool / Material/base material | Silane coupling | Adsorption (wt%) | Halo width before wash | Halo width after wash |
|---|---|---|---|---|---|---|
| Com. Ex. 1-1 | (5) | Pile puff / Cotton velvet | Not used | 0.11 | 2 mm | 0 mm |
| | | | Used | 0.15 | 3 | 0 |
| Com. Ex.1-2 | (5) | Pile puff / Nylon tricot pile | Not used Used | 0.10 | 1 | 0 |
| | | | | 0.14 | 2 | 0 |
| Com. Ex. 1-3 | (6) | Eyelash / Horse hair | Not used Used | 0.16 | 1 | 0 |
| | | | | 0.12 | 2 | 0 |
| Com. Ex. 1-4 | (7) | Wig / Acrylic wig | Not used | 0.70 | 4 | 0 |
| | | | Used | 1.40 | 4 | 2 |
| Com. Ex. 1-5 | (8) | Porous puff / Polyurethane sponge | Not used | 0.80 | 4 | 0 |
| | | | Used | 1.50 | 4 | 3 |
| Com. Ex. 1-6 | (9) | Porous tip / NBR sponge | Not used | 0.60 | 5 | 1 |
| | | | Used | 1.50 | 5 | 2 |
| Com. Ex. 1-7 | (10) | Pile puff / PET circular knit pile | Not used | 0.60 | 5 | 1 |
| | | | Used | 1.80 | 5 | 2 |

When the results shown in Table 2 (examples of the present invention) are compared with those shown in Table 3 (comparative examples), the puffs made in accordance with the present invention, which contain both constituents (A) and (B), clearly exhibit a halo effect after wash and provide an antibacterial property resistant to wash. On the other hand, it is evident that the products containing either constituent (A) or (B), but not both, do not offer wash resistance.

### Example 2

A brush material was soaked in each of the processing solutions shown in Table 1, based on the solution volume of 15 times the weight of the brush material (bath ratio of 1:15). The solution was heated to 96°C for 60 minutes, after which the material was washed in water and then dried.

As the silane coupling, an aqueous solution containing γ-methacryloxypropyl trimethoxysilane (product number SH6030, manufactured by Toray Silicone) by 1 percent by weight was prepared and processed at 70°C for 60 minutes.

The halo test against bacteria in the brush material was conducted using Staphylococcus Aureus in accordance with JIS-L-1902.

Next, the wash-resistance test was conducted in accordance with JIS-L-1042, where 100 hand washes by rubbing following a 30-minute soak in a 40°C wash-test solution were repeated twice, after which the material was washed in water and then dried.

A total of four brush materials were used in the tests. They are: (1) horse hair, (2) nylon, (3) PET, and (4) mixture of horse hair and PET.

The halo test results are shown in Tables 4 and 5.

**Table 4**

| Example | Processing solution | Brush material | Silane coupling | Adsorption (wt%) | Halo width before wash | Halo width after wash |
|---|---|---|---|---|---|---|
| Ex. 2-1 | (1) | Horse hair | Not used | 0.7 | 7 mm | 7 mm |
| | (1) | Horse hair | Used | 1.7 | 8 | 8 |
| Ex. 2-2 | (2) | Horse hair | Not used | 1.5 | 8 | 8 |
| | (2) | Horse hair | Used | 2.6 | 9 | 9 |
| Ex. 2-3 | (3) | Horse hair | Not used | 0.8 | 7 | 7 |
| | (3) | Horse hair | Used | 2.3 | 9 | 9 |
| Ex. 2-4 | (4) | Horse hair | Not used | 1.7 | 8 | 8 |
| | (4) | Horse hair | Used | 2.7 | 9 | 9 |
| Ex. 2-5 | (1) | Nylon | Not used | 0.7 | 7 | 7 |
| | (1) | Nylon | Used | 1.6 | 8 | 8 |
| Ex. 2-6 | (2) | Nylon | Not used | 0.9 | 7 | 7 |
| | (2) | Nylon | Used | 2.0 | 8 | 8 |
| Ex. 2-7 | (3) | Nylon | Not used | 0.8 | 7 | 7 |
| | (3) | Nylon | Used | 1.7 | 8 | 8 |
| Ex 2-8 | (4) | Nylon | Not used | 1.0 | 7 | 7 |
| | (4) | Nylon | Used | 2.0 | 8 | 8 |
| E 2-9 | (1) | PET | Not used | 0.4 | 6 | 6 |
| | (1) | PET | Used | 1.1 | 7 | 7 |
| Ex. 2-10 | (2) | PET | Not used | 0.5 | 6 | 6 |
| | (2) | PET | Used | 1.7 | 8 | 8 |
| Ex. 2-11 | (3) | PET | Not used | 0.6 | 6 | 6 |
| | (3) | PET | Used | 1.3 | 7 | 7 |
| Ex. 2-12 | (4) | PET | Not used | 0.6 | 6 | 6 |
| | (4) | PET | Used | 1.7 | 8 | 8 |
| Ex. 2-13 | (1) | Horse hair + PET | Not used | 0.5 | 6 | 6 |
| | (1) | Horse hair + PET | Used | 1.4 | 7 | 7 |
| Ex. 2-14 | (2) | Horse hair + PET | Not used | 0.6 | 6 | 6 |
| | (2) | Horse hair + PET | Used | 1.8 | 8 | 8 |
| Ex. 2-15 | (3) | Horse hair + PET | Not used | 0.6 | 6 | 6 |
| | (3) | Horse hair + PET | Used | 1.5 | 7 | 7 |
| Ex. 2-16 | (4) | Horse hair + PET | Not used | 0.5 | 6 | 6 |
| | (4) | Horse hair + PET | Used | 1.9 | 8 | 8 |

**Table 5**

| Comparative example | Processing solution | Brush material | Silane coupling | Adsorption (wt%) | Halo width before wash | Halo width after wash |
|---|---|---|---|---|---|---|
| Com. Ex. 2-1 | (5) | Horse hair | Not used | 0.05 | 2 mm | 0 mm |
| | (5) | Horse hair | Used | 0.12 | 3 | 0 |
| Com. Ex. 2-2 | (5) | PET | Not used | 0.08 | 1 | 0 |
| | (5) | PET | Used | 0.15 | 2 | 0 |
| Com. Ex. 2-3 | (6) | PET | Not used | 0.10 | 1 | 0 |
| | (6) | PET | Used | 0.16 | 2 | 0 |
| Com. Ex. 2-4 | (7) | PET | Not used | 0.70 | 6 | 0 |
| | (7) | PET | Used | 1.40 | 6 | 2 |
| Com. Ex. 2-5 | (8) | PET | Not used | 0.80 | 6 | 0 |
| | (8) | PET | Used | 1.50 | 6 | 3 |
| Com. Ex. 2-6 | (9) | PET | Not used | 0.60 | 6 | 1 |
| | (9) | PET | Used | 1.50 | 6 | 2 |
| Com. Ex. 2-7 | (10) | PET | Not used | 0.60 | 6 | 1 |
| | (10) | PET | Used | 1.80 | 6 | 2 |

When the results shown in Table 4 (examples of the present invention) are compared with those shown in Table 5 (comparative examples), the brushes made in accordance with the present invention, which contain both constituents (A) and (B), clearly exhibit a halo effect after wash and provide an antibacterial property resistant to wash. On the other hand, it is evident that the products containing either constituent (A) or (B), but not both, do not offer wash resistance.

The present application claims priority to Japanese Patent Application No. 2004-120600, filed April 15, 2004, No. 2004-157337, filed May 27, 2004, and No. 2004-157351, filed May 27, 2004, the disclosure of which is incorporated herein by reference in their entirety.

## Claims

1. A method for producing a cosmetic tool having an antibacterial property, comprising:
providing a solution containing 0.1-5.0% by weight of (A) benzyl ammonium chloride compound and 0.1-5.0% by weight of (B) naphthoquinone compound;
soaking a base material for a cosmetic tool in the solution, said material being selected from the group consisting of raised woven fabric, raised knit fabric, nonwoven pile, flocked fabric, monofilament, animal hair, synthetic fiber, wig material, polyurethane sponge, and NBR sponge; and
subjecting the base material in the solution to hot solution processing to absorb the compounds (A) and (B) to the base material to provide the base material with an antibacterial halo width of 5 mm or more maintained after hand wash.

2. The method as described in Claim 1, wherein a silane coupling agent is included in the solution and the compounds (A) and (B) are adsorbed to the base material via the silane coupling agent.

3. The method as described in Claim 1 or 2, further comprising producing a cosmetic tool using the base material, which is selected from the group consisting of a cosmetic brush, pile puff, porous puff, porous tip, flocked tip, eyelash and wig.

4. The method as described in any one of Claims 1 to 3, wherein the synthetic fiber is at least one or more fibers selected from the group consisting of nylon, PBT (polybutylene terephthalate), PET (polyethylene terephthalate), acrylic and modacrylic; or a sheath-core fiber having a surface layer formed by hydrophilic polymer and an inside formed by hydrophobic polymer.

5. The method as described in any one of Claims 1 to 4, wherein the hot solution processing is conducted at a temperature of 80°C or higher.

6. The method as described in any one of Claims 1 to 5, which is conducted simultaneously with dyeing of the base material using the solution containing a dye.

7. The method as described in any one of Claims 1 to 6, wherein the solution further contains a carrier agent.

8. The method as described in Claim 7, wherein the carrier agent is selected from the group consisting of benzoic acid, methyl p-hydroxybenzoate, ethyl p-hydroxybenzoate, butyl p-hydroxybenzoate, bisphenol A, bisphenol B, 2-hydroxybisphenol, p-biphenylamine, p-aminosalicylic acid, and orthophenyl phenol.

9. A cosmetic tool having an antibacterial property with an antibacterial halo width of 5 mm or more maintained even after hand wash by rubbing, obtainable by causing 0.1 to 5.0 percent by weight of (A) a benzyl ammonium chloride compound and 0.1 to 5.0 percent by weight of (B) a naphthoquinone compound to be adsorbed, through hot solution processing, to the cosmetic tool.

10. A cosmetic tool as described in Claim 9, obtainable by causing (A) a benzyl ammonium chloride compound and (B) a naphthoquinone compound to be adsorbed, through hot solution processing and via a silane coupling agent, to the cosmetic tool.

11. The cosmetic tool as described in Claim 9 or 10, wherein the cosmetic tool is selected from a cosmetic brush, pile puff, porous puff, porous tip, flocked tip, eyelash or wig.

12. The cosmetic tool as described in any one of Claims 9 to 11, wherein the base material of the cosmetic tool is selected from animal hair, synthetic fiber, monofilament, raised woven fabric, raised knit fabric, nonwoven pile, flocked fabric, wig material, polyurethane sponge or NBR sponge.

13. The cosmetic tool as described in Claim 12, wherein the synthetic fiber is selected from at least one or more fibers selected from the group consisting of nylon, PBT (polybutylene terephthalate), PET (polyethylene terephthalate), acrylic and modacrylic; or a sheath-core fiber having a surface layer formed by hydrophilic polymer and an inside formed by hydrophobic polymer.

14. A method for producing a cosmetic tool having an antibacterial property with an antibacterial halo width of 5 mm or more maintained even after hand wash by rubbing, **characterized by** soaking the cosmetic tool in an aqueous solution containing (A) a benzyl ammonium chloride compound and (B) a naphthoquinone compound, and applying hot solution processing.

15. The method for producing a cosmetic tool as described in Claim 14, wherein the base material of the cosmetic tool is selected from animal hair, synthetic fiber, monofilament, raised woven fabric, raised knit fabric, nonwoven pile, flocked fabric, wig material, polyurethane sponge or NBR sponge.

16. The method for producing a cosmetic tool as described in Claim 14 or 15, **characterized by** applying hot solution processing to the cosmetic tool in an aqueous solution containing (A) a benzyl ammonium chloride compound and (B) a naphthoquinone compound to which a carrier is added.

17. The method for producing a cosmetic tool as described in Claim 16, wherein as the carrier benzoic acid, methyl p-hydroxybenzoate, ethyl p-hydroxybenzoate, butyl p-hydroxybenzoate, bisphenol A, bisphenol B, 2-hydroxybisphenol, p-biphenylamine, p-aminosalicylic acid, orthophenyl phenol, or cyanoethylbenzyl ether is used.
